(19) Europäisches Patentamt
European Patent Office
Office européen des brevets

(11) Veröffentlichungsnummer: **0 302 265**
**A1**

(12) # EUROPÄISCHE PATENTANMELDUNG

(21) Anmeldenummer: **88111186.8**

(51) Int. Cl.4: **A61K 7/09 , A61K 7/155**

(22) Anmeldetag: **13.07.88**

(30) Priorität: **07.08.87 DE 3726230**

(43) Veröffentlichungstag der Anmeldung:
**08.02.89 Patentblatt 89/06**

(84) Benannte Vertragsstaaten:
**AT BE CH DE ES FR GB GR IT LI LU NL SE**

(71) Anmelder: **Wella Aktiengesellschaft**
**Berliner Allee 65**
**D-6100 Darmstadt(DE)**

(72) Erfinder: **Greiche, Youssef, Dr.**
**Soderstrasse 36**
**D-6100 Darmstadt(DE)**
Erfinder: **Hartmann, Peter**
**Hoffmannstrasse 6**
**D-6100 Darmstadt(DE)**
Erfinder: **Köhler, Joachim, Dr.**
**Waldstrasse 51**
**D-6101 Brensbach/Odenwald(DE)**

(54) **Mittel und Verfahren zur dauerhaften Verformung oder Entfernung von Haaren.**

(57) Gegenstand der Erfindung ist ein Dimethylisosorbit enthaltendes Mittel zur dauerhaften Verformung oder Entfernung von Haaren auf der Basis einer keratinreduzierenden Mercaptoverbindung. Die Erfindung betrifft ebenfalls ein Verfahren zur dauerhaften Verformung oder Entfernung von Haaren unter Verwendung dieses Mittels.

Das erfindungsgemäße kosmetische Mittel ist frei von störendem Mercaptan- oder Ammoniakgeruch und besitzt eine hohe Verformungswirksamkeit

EP 0 302 265 A1

## Mittel und Verfahren zur dauerhaften Verformung oder Entfernung von Haaren

Die vorliegende Erfindung betrifft ein Dimethylisosorbit enthaltendes Mittel zur dauerhaften Verformung oder Entfernung von Haaren sowie ein Verfahren zur dauerhaften Verformung oder Entfernung von Haaren unter Verwendung dieses Mittels.

Für die dauerhafte Haarverformung werden üblicherweise Verformungsmittel verwendet, die als keratinreduzierenden Wirkstoff eine Schwefelverbindung, beispielsweise Ammoniumthioglykolat, Ammoniumthiolactat, 3-Mercaptopropionsäure, Cystein, Cysteamin oder Monothioglykolsäureglycerinester, enthalten. Weiterhin können derartige Mittel zur pH-Einstellung Ammoniak sowie Ammoniumcarbonat oder -hydrogencarbonat enthalten.

Die chemische Entfernung von Haaren ("Depilation") erfolgt in ähnlicher Weise wie die dauerhafte Haarverformung durch die Behandlung der Haare mit einer keratinreduzierenden Mercaptoverbindung. Infolge eines stärker alkalischen pH-Wertes (pH = 11 bis 13) verläuft bei der Depilation der Prozeß der Quellung des Haares und die Spaltung der Disulfidbrückenbindungen des Haarkeratins jedoch bis zur völligen Auflösung der Haare.

Die vorstehend beschriebenen Mittel zur dauerhaften Verformung oder Entfernung von Haaren besitzen aufgrund ihres Gehaltes an Mercaptoverbindungen und Ammoniak einen unangenehmen und stechenden Geruch.

Während der Lagerung von derartigen Mitteln kommt es zudem zur Bildung von Schwefelwasserstoff, wodurch der penetrante Mercaptangeruch dieser Präparate noch verstärkt wird.

Ein weiteres Problem besteht darin, daß das Haar auch nach Beendigung der Verformungsbehandlung einen störenden Mercaptangeruch besitzt. Insbesondere bei Verwendung von Verformungsmitteln auf der Basis von Thiolactat, Cystein oder Glycerinmonothioglykolat ist dieser Mercaptangeruch noch einige Stunden, manchmal auch Tage, nach der Verformungsbehandlung im Haar feststellbar.

Es wurde bereits eine Vielzahl von Versuchen unternommen, um den unangenehmen Geruch derartiger Präparate zu beseitigen.

So wurde beispielsweise versucht, den stechenden Ammoniakgeruch durch Verwendung von bestimmten; relativ geruchsarmen Aminen, wie zum Beispiel Monoethanolamin, an Stelle von Ammoniak zu beseitigen. Der Ersatz von Ammoniak durch derartige Amine führt jedoch zu einer deutlichen Verschlechterung der physiologischen Verträglichkeit der Präparate und ist daher wenig sinnvoll.

Weiterhin wurde versucht, den unangenehmen Eigengeruch von Verformungsmitteln durch Zusatz von Parfümölen, insbesondere solchen mit einer frisch-fruchtig wirkenden Note, zu überdecken. Der Zusatz von derartigen Parfümölen führt aber ebenfalls nicht zu zufriedenstellenden Ergebnissen da die Parfümöle nur den Ammoniakgeruch, nicht jedoch den äußerst unangenehmen Mercaptangeruch, in ausreichendem Maße zu überdecken vermögen.

Ebenfalls wurde es bereits versucht, durch den Zusatz bestimmter Verbindungen eine Geruchsstabilisierung von mercaptanhaltigen Verformungsmitteln zu erzielen.

So wird beispielsweise in der **GB-PS 674 195** (W. FREUND) empfohlen, mercaptanhaltigen Verformungsmitteln zur Geruchsstabilisierung Metallamminsalze, insbesondere Zinktetramminsalze, zuzusetzen.

Ein derartiger Zusatz kann jedoch zu unerwünschten Metallbelastungen sowie zur Verfärbung der Haare führen.

Durch die in der **DE-OS 32 43 959** (E.MERCK) empfohlene Verwendung von Ascorbinsäure, Ascorbinsäuresalzen, 2-Keto-L-gulonsäure, mehrwertigen Phenolen oder Methenamin zur Geruchsstabilisierung von thioglykolsäurehaltigen Präparaten wird ebenfalls kein befriedigendes Ergebnis erzielt, da durch die zur Geruchsstabilisierung zugesetzten Verbindungen die Wirksamkeit des Verformungsmittels herabgesetzt wird.

Auch wurde in der Literatur bereits vorgeschlagen, bei Mercaptoverbindungen enthaltenden kosmetischen Mitteln die Geruchsbelästigung durch den Zusatz spezieller Deodorantien zu beseitigen. So empfiehlt beispielsweise die **offengelegte japanische Patentanmeldung 56 - 7424** (SUN STAR HAMAGAKIK), mercaptanhaltigen Präparaten zur Desodorierung aliphatische Triglyceride mit einer Alkylkettenlänge von 6 bis 8 Kohlenstoffatomen zuzusetzen, während die **offengelegte japanische Patentanmeldung 56 - 7436** (KAO SOAP KK) zur Desodorierung derartiger Präparate den Zusatz bestimmter Styrolderivate empfiehlt. Weiterhin ist aus der **DE-OS 32 33 664** (KAO CO.) ein Mittel zur Geruchsbeseitigung und Desodorierung bekannt, welches als Hauptwirkstoff ein spezielles Keratinhydrolysat enthält. Dieses Mittel soll insbesondere für die Beseitigung des von Mercaptanen hervorgerufenen unangenehmen Geruches geeignet sein.

Eine Verwendung der in den drei zuletzt aufgeführten Schriften zur Beseitigung des störenden Mercaptangeruches empfohlenen Deodorantien in Mitteln zur dauerhaften Verformung oder Entfernung von

Haaren ist jedoch nicht empfehlenswert, da derartige Verbindungen zu einer Herabsetzung der Wirksamkeit dieser Mittel und damit zu einem unbefriedigenden Behandlungsergebnis führen.

Es bestand daher die Aufgabe, den durch Mercaptoverbindungen und Ammoniak hervorgerufenen unangenehmen Geruch von Mitteln zur dauerhaften Verformung oder Entfernung von Haaren zu beseitigen, ohne hierdurch die Wirkung und die physiologische Verträglichkeit dieser Mittel zu verschlechtern.

Überraschenderweise wurde nun gefunden, daß der Zusatz von Dimethylisosorbit zu Mercaptoverbindungen enthaltenden Mitteln zur dauerhaften Verformung oder Entfernung von Haaren eine wirksame Beseitigung sowohl des Ammoniak- als auch des Mercaptangeruches ermöglicht, ohne daß die Wirkung und die physiologische Verträglichkeit dieser Mittel negativ beeinflußt wird.

Gegenstand der vorliegenden Erfindung ist daher ein kosmetisches Mittel zur dauerhaften Verformung oder Entfernung von Haaren auf der Basis einer keratinreduzierenden Mercaptoverbindung, welches dadurch gekennzeichnet ist, daß es Dimethylisosorbit der Formel (I)

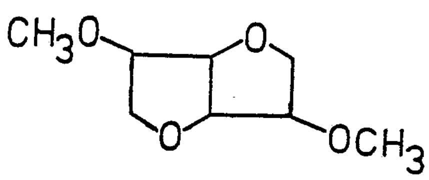

(I)

enthält.

Das erfindungsgemäße kosmetische Mittel kann in Form eines Mittels zur dauerhaften Haarverformung (Dauerwellung oder Entkräuselung) oder eines Mittels zur Entfernung der Haare vorliegen. Die Zusammensetzung dieser Mittel entspricht den für derartige Mittel jeweils bekannten Zubereitungsarten auf der Basis von keratinreduzierenden Mercaptoverbindungen. Das Dimethylisosorbit der Formel (I) ist in diesen Mitteln vorzugsweise in einer Menge von 0,01 bis etwa 5 Gewichtsprozent (bezogen auf die Gesamtmenge der gebrauchsfertigen Zubereitung) enthalten.

Diese üblichen Zubereitungsarten, die im Rahmen der Erfindung verwendbar sind, sollen nachstehend erläutert werden.

Bei dem Mittel zur dauerhaften Haarverformung, und zwar sowohl zur Dauerwellung als auch zur Entkräuselung, handelt es sich insbesondere um eine wäßrige, alkalisch (pH = 7 bis 10) eingestellte Zubereitung, welche eine keratinreduzierende Mercaptoverbindung, wie zum Beispiel Cystein, Cysteamin, N-Acetyl-L-cystein, Mercaptocarbonsäuren, beispielsweise Thioglykolsäure oder Thiomilchsäure,oder Salze von Mercaptocarbonsäuren, wie zum Beispiel Ammonium- und Guanidinsalze der Thioglykolsäure oder Thiomilchsäure, in einer Konzentration von etwa 5 bis 12 Gewichtsprozent enthält. Die erforderliche Alkalität wird hierbei durch die Zugabe von Ammoniak, organischen Aminen, Ammonium- und Alkalicarbonat oder -hydrogencarbonat eingestellt. Es kommt aber auch ein neutral oder sauer eingestelltes Haarverformungsmittel (pH = 4,5 bis 7,0) in Betracht, das in wäßrigem Medium einen wirksamen Gehalt an Sulfiten oder Mercaptocarbonsäureestern aufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als $SO_2$) verwendet. Im letzteren Fall kommen insbesondere Monothioglykolsäureglykolester oder -glycerinester in einer Konzen tration von etwa 5 bis 30 Gewichtsprozent (entsprechend einem Gehalt an freier Thioglykolsäure von 2 bis 16 Gewichtsprozent) zur Anwendung.

In dem Mittel zur dauerhaften Haarverformung kann auch ein Gemisch der vorstehend genannten keratinreduzierenden Mercaptoverbindungen enthalten sein.

Handelt es sich bei dem erfindungsgemäßen Mittel um ein Mittel zur Entfernung der Haare, so liegt es als wäßrige, stark alkalische (pH = 11 bis 12,5) Zubereitung auf der Basis einer keratinreduzierenden Mercaptoverbindung vor. Als keratinreduzierende Mercaptoverbindung enthält ein derartiges Mittel vorzugsweise eine Verbindung aus der Gruppe der Sulfide, wie zum Beispiel Strontiumsulfid, oder ein Salz einer Mercaptocarbonsäure, wie beispielsweise Natrium- und Calciumthioglykolat oder Natrium- und Calciumthiolactat, in einer Menge von etwa 5 bis 10 Gewichtsprozent. Zur Einstellung des erforderlichen pH-Wertes verwendet man hierbei vorzugsweise Calcium- oder Strontiumhydroxid sowie Calcium- oder Magnesiumcarbonat in einer Menge von etwa 1 bis 25 Gewichtsprozent.

Das erfindungsgemäße Mittel zur dauerhaften Verformung oder Entfernung von Haaren kann in Form einer wäßrigen Lösung oder Emulsion sowie in verdickter Form auf wäßriger Basis, insbesondere als Creme, Gel oder Paste vorliegen Ebenfalls ist es möglich, dieses Mittel gemeinsam mit einem Treibgas

unter Druck in Aerosoldosen abzufüllen und daraus als Schaum zu entnehmen.

Selbstverständlich kann das erfindungsgemäße Mittel zur dauerhaften Haarverformung oder zur Entfernung von Haaren alle für ein derartiges Mittel üblichen und bekannten Zusatzstoffe, zum Beispiel Verdickungsmittel, wie beispielsweise Kaolin, Bentonit, Fettsäuren, höhere Fettalkohole, Stärke, Polyacrylsäure und deren Derivate, Cellulosederivate, Alginate, Vaseline oder Paraffinöl, Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quartäre Ammoniumsalze, Alkylbetaine, oxethylierte Fettalkohole, oxethylierte Alkylphenole, Fettsäurealkanolamide oder oxethylierte Fettsäureester, ferner Trübungsmittel, wie zum Beispiel Polyethylenglykolester, oder Alkohole, wie beispielsweise Ethanol, Propanol, Isopropanol oder Glycerin, Lösungsvermittler, Stabilisatoren, Puffersubstanzen, Parfümöle, Farbstoffe sowie haarkonditionierende und haarpflegende Bestandteile, wie zum Beispiel kationische Polymere, Lanolinderivate, Cholesterin, Pantothensäure oder Betain, enthalten. Die erwähnten Bestandteile werden in den für solche Zwecke üblichen Mengen verwendet, zum Beispiel die Netzmittel und Emulgatoren in Konzentrationen von etwa 0,2 bis 10 Gewichtsprozent, während die Verdickungsmittel in einer Menge von etwa 1,0 bis 25 Gewichtsprozent in diesem Mittel enthalten sein können.

Weiterhin können diesem Mittel zusätzlich bekannte wirkungsverstärkende Stoffe, wie zum Beispiel Dipropylenglykolmonomethylether oder 2-Pyrrolidon, in einer Menge von etwa 2 bis 30 Gewichtsprozent zugesetzt werden.

Das erfindungsgemäße, Dimethylisosorbit enthaltende, Mittel zur dauerhaften Verformung oder Entfernung von Haaren auf der Basis einer keratinreduzierenden Mercaptoverbindung besitzt im Gegensatz zu üblichen Mitteln auch nach längerer Lagerung einen angenehmen frischen Geruch und ist, wie eine Überprüfung mit Bleiacetatpapier zeigt, frei von Schwefelwasserstoff. Bei der Anwendung des erfindungsgemäßen Mittels ist ebenfalls kein störender Mercaptangeruch auf den Haaren oder der Haut feststellbar.

Die Wirksamkeit dieses Präparates wird durch den Zusatz von Dimethylisosorbit nicht beeinträchtigt; vielmehr besitzt das erfindungsgemäße Mittel im Vergleich zu üblichen Verformungsmitteln eine gesteigerte Verformungswirkung.

Da das in dem erfindungsgemäßen Mittel zur Geruchsstabilisierung enthaltene Dimethylisosorbit chemisch inert und pH-stabil sowie ungiftig ist, wird die physiologische Verträglichkeit des Mittels ebenfalls nicht negativ beeinflußt.

Die vorliegende Erfindung betrifft weiterhin ein Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, anschließend zur Frisur legt und sodann trocknet, welches dadurch gekennzeichnet ist, daß für die Verformungsbehandlung das vorstehend beschriebene, Dimethylisosorbit enthaltende, Verformungsmittel verwendet wird.

Bei einer Ausführungsform des erfindungsgemäßen Verfahrens wird das Haar zunächst mit einem Shampoo gewaschen und danach mit Wasser gespült. Anschließend wird das handtuchtrockene Haar in einzelne Strähnen aufgeteilt und auf Wickler gewickelt. Der Durchmesser der Wickler beträgt hierbei, je nachdem ob eine Dauerwellung oder eine Haarentkräuselung gewünscht wird, etwa 5 bis 13 Millimeter oder etwa 15 bis 35 Millimeter. Sodann wird das Haar mit einer für die Haarverformung ausreichenden Menge, vorzugsweise etwa 80 bis 120 Gramm, des erfindungsgemäßen Verformungsmittels behandelt.

In einer weiteren Ausführungsform des erfindungsgemäßen Verfahrens wird das Haar im Anschluß an die Haarwäsche mit einem Teil des Verformungsmittels, vorzugsweise etwa 40 bis 60 Gramm, vorgefeuchtet, in einzelne Strähnen aufgeteilt und auf Wickler gewickelt. Sodann wird das Haar mit dem restlichen Verformungsmittel, vorzugsweise etwa 40 bis 60 Gramm, nochmals behandelt.

Nach einer für die dauerhafte Verformung des Haares ausreichenden Einwirkungszeit, welche je nach Haarbeschaffenheit, dem pH-Wert und der Verformungswirksamkeit des Verformungsmittels sowie in Abhängigkeit von der Anwendungstemperatur etwa 5 bis 45 Minuten (5 bis 30 Minuten mit Wärmeeinwirkung; 20 bis 45 Minuten ohne Wärmeeinwirkung) beträgt, wird das Haar mit Wasser gespült und anschließend oxidativ nachbehandelt ("fixiert"). Das Nachbehandlungsmittel wird, je nach Haarfülle, in einer Menge von etwa 80 bis 100 Gramm verwendet.

Für die oxidative Nachbehandlung kann jedes beliebige, bisher für eine derartige Behandlung verwendete, Nachbehandlungsmittel verwendet werden. Beispiele für in einem derartigen Nachbehandlungsmittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Normalerweise liegt das Oxidationsmittel in dem wäßrigen Nachbehandlungsmittel in einer Konzentration von etwa 0,5 bis 10 Gewichtsprozent vor.

Das Mittel für die oxidative Nachbehandlung kann selbstverständlich weitere Stoffe, wie zum Beispiel Netzmittel, schwache Säuren, Puffersubstanzen oder Peroxidstabilisatoren, enthalten und sowohl in Form einer wäßrigen Lösung oder Emulsion als auch in verdickter Form auf wäßriger Basis, insbesondere als Creme, Gel oder Paste, vorliegen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet. Im Falle einer Haarentkräuselung kann auch so verfahren werden, daß man das oxidierend wirkende Nachbehandlungsmittel bei gewickeltem Haar mit Wasser abspült und das Haar anschließend, ohne abzuwickeln, direkt am Wickler trocknet.

Bei der Haarentkräuselung ist es ebenfalls möglich das Haar ohne Anwendung von Wicklern durch mehrmaliges Kämmen während der Einwirkungszeit zu glätten. Anschließend wird das Haar mit lauwarmem Wasser gründlich gespült und oxidativ nachbehandelt.

Ein weiterer Gegenstand der vorliegenden Erfindung ist ein Verfahren zur Entfernung von Haaren ("Depilation"), bei dem man ein Enthaarungsmittel auf die zu behandelnden Stellen aufbringt, das Enthaarungsmittel nach einer kurzen Einwirkungszeit wieder entfernt und anschließend mit lauwarmem Wasser nachspült, welches dadurch gekennzeichnet ist, daß für die Enthaarungsbehandlung das vorstehend beschriebene, Dimethylisosorbit enthaltende, Mittel verwendet wird.

Für die Entfernung von Haaren wird das erfindungsgemäße Mittel, zweckmäßigerweise in Form einer Creme oder Paste, mit einem Spatel auf das zu behandelnde Haar so aufgetragen, daß die Haare von dem Enthaarungsmittel vollständig bedeckt werden. Nach einer kurzen Einwirkungszeit, vorzugsweise nach etwa 3 bis 10 Minuten, wird das Enthaarungsmittel mit einem Spatel oder einem Wattebausch entfernt und die Haut mit lauwarmem Wasser gründlich abgespült.

Die nachfolgenden Beispiele sollen den Gegenstand der Erfindung näher erläutern, ohne ihn hierauf zu beschränken.

## Beispiele

**Beispiel 1:** Thioglykolathaltiges Dauerwellmittel

| | |
|---|---|
| 1,0 g | Dimethylisosorbit |
| 11,5 g | Ammoniumthioglykolat |
| 4,0 g | Ammoniumcarbonat |
| 1,0 g | Kokosfettsäuredimethylammoniumbetain |
| 0,3 g | Parfümöl |
| 82,2 g | Wasser, vollentsalzt |
| 100,0 g | |

Der pH-Wert dieses Mittels beträgt 8,7.

Das Haar wird mit einem milden Shampoo gewaschen. Anschließend wird das handtuchtrockene Haar mit etwa der Hälfte der vorstehend aufgeführten Wellflüssigkeit vorgefeuchtet, auf Dauerwellwickler mit einem Durchmesser von 8 Millimetern gewickelt und sodann mit der restlichen Dauerwellflüssigkeit nachgefeuchtet. Anschließend werden die Haare mit einer Plastikfolie abgedeckt. Nach einer Einwirkungszeit von 20 Minuten wird die Abdeckung entfernt, das gewickelte Haar mit Wasser gründlich gespült und sodann mit 80 Gramm einer 3-prozentigen Hydrogenperoxidlösung oxidativ nachbehandelt. Nach Entfernung der Wickler werden die Haare erneut mit Wasser gespült und anschließend zur Frisur gelegt und getrocknet.

Das so behandelte Haar besitzt eine gleichmäßige Krause und einen angenehmen, frischen Geruch.

| | |
|---|---|
| 2,0 g | Dimethylisosorbit |
| 12,0 g | Thiomilchsäure |
| 10,0 g | Ammoniak, 25-prozentige wäßrige Lösung |
| 0,5 g | Parfümöl |
| 75,5 g | Wasser, vollentsalzt |
| 100,0 g | |

**Beispiel 2:** Dauerwellmittel auf Thiomilchsäurebasis

Der pH-Wert dieses Mittels beträgt 9,3.
Die Anwendung dieses Dauerwellmittels erfolgt in der im Beispiel 1 beschriebenen Weise.

**Beispiel 3:** Verformungsmittel auf Cysteinbasis

| | |
|---|---|
| 3,0 g | Dimethylisosorbit |
| 6,0 g | Cystein |
| 2,5 g | Monoethanolamin |
| 0,5 g | Parfümöl |
| 88,0 g | Wasser, vollentsalzt |
| 100,0 g | |

Der pH-Wert des Verformungsmittels beträgt 9,2.
Das gewaschene und handtuchtrockene Haar wird auf Wickler mit einem Durchmesser von 7 Millimetern gewickelt und anschließend mit dem obigen Verformungsmittel gut durchfeuchtet Sodann wird das Haar mit einer Plastikhaube abgedeckt und 25 Minuten lang unter einer Trockenhaube bei einer Temperatur von 40 bis 45 Grad Celsius erwärmt Anschließend wird die Abdeckung entfernt, das Haar mit Wasser gespült und sodann mi 100 Gramm einer 3-prozentigen Hydrogenperoxidlösung oxidativ nachbehandelt. Nach Entfernung der Wickler werden die Haare erneut mit Wasser gespült, zur Wasserwelle gelegt und sodann getrocknet.

**Beispiel 4:** Cysteaminhaltiges Verformungsmittel

| | |
|---|---|
| 0,5 g | Dimethylisosorbit |
| 10,0 g | Cysteamin |
| 5,0 g | Ammoniumcarbonat |
| 0,2 g | Parfümöl |
| 84,3 g | Wasser, vollentsalzt |
| 100,0 g | |

Das Mittel besitzt einen pH-Wert von 8,8.
Das Haar wird mit einem Shampoo gewaschen und mit Wasser gründlich gespült. Anschließend wird etwa die Hälfte des Haarverformungsmittels gleichmäßig auf dem frottierten Haar verteilt, das Haar auf Wickler mit einem Durchmesser von 10 Millimetern gewickelt und mit dem restlichen Verformungsmittel nochmals befeuchtet. Sodann wird das Haar 15 Minuten lang mit einem Wärmestrahler auf 45 Grad Celsius erwärmt. Anschließend wird das Haar mit Wasser gespült und mit 100 Gramm einer 2-prozentigen Hydrogenperoxidlösung oxidativ nachbehandelt. Nach Entfernung der Wickler werden die Haare erneut mit Wasser gespült und danach getrocknet.

**Beispiel 5:** Saures Dauerwellmittel auf Thioglykolsaüreesterbasis

| | | |
|---|---|---|
| Komponente 1: | 2,5 g<br>37,5 g<br>10,0 g<br>50,0 g | Dimethylisosorbit<br>Thioglykolsäuremonoglycerinester<br>Glycerin |
| Komponente 2: | 3,0 g<br>0,6 g<br>0,5 g<br>95,9 g<br>100,0 g | Dipropylenglykolmonomethylether<br>Parfümöl<br>Ammoniumhydrogencarbonat<br>Wasser, vollentsalzt |

Unmittelbar vor dem Gebrauch werden 30 Gramm der Komponente 1 mit 70 Gramm der Komponente 2 zu einem gebrauchsfertigen Dauerwellmittel (pH = 5,8) vermischt.

Im Anschluß an eine Haarwäsche wird das handtuchtrockene Haar mit etwa der Hälfte dieses Dauerwellmittels gleichmäßig durchfeuchtet und sodann auf Wickler mit einem Durchmesser von 6 Millimetern gewickelt. Anschließend wird das Haar mit dem restlichen Dauerwellmittel nochmals befeuchtet. Nach einer Einwirkungszeit von 20 bis 25 Minuten bei 40 Grad Celsius wird das Haar mit Wasser gespült und sodann mit 100 Gramm einer 3-prozentigen Hydrogenperoxidlösung oxidativ nachbehandelt. Nach Entfernung der Wickler werden die Haare erneut mit Wasser gespült und dann zur Wasserwelle gelegt und getrocknet.

Das Haar besitzt einen angenehmen Geruch und eine gleichmäßige Krause.

**Beispiel 6:** Entkräuselungscreme auf Thioglykolatbasis

| | |
|---|---|
| 1,5 g | Dimethylisosorbit |
| 20,0 g | Ammoniumthioglykolat, 50-prozentige wäßrige Lösung |
| 6,0 g | Cetylalkohol |
| 4,5 g | Oleylalkohol, mit 20 Mol Ethylenoxid oxethyliert |
| 4,5 g | Dipropylenglykolmonomethylether |
| 3,2 g | Ammoniak, 25-prozentige wäßrige Lösung |
| 1,0 g | Paraffinöl |
| 0,5 g | kolloidale Kieselsäure |
| 0,3 g | Parfümöl |
| 58,5 g | Wasser, vollentsalzt |
| 100,0 g | |

Der pH-Wert dieser Entkräuselungscreme beträgt 9,5.

Die obige Entkräuselungscreme wird auf das Haar aufgetragen und gleichmäßig verteilt. Während der Einwirkungszeit der Entkräuselungscreme, welche etwa 10 Minuten beträgt, wird das Haar mehrmals glattgekämmt. Sodann wird das Haar mit Wasser gründlich gespült und anschließend mit 100 Gramm einer 5-prozentigen Natriumbromatlösung oxidativ nachbehandelt. Danach wird das Haar erneut mit Wasser gespült und anschließend getrocknet.

**Beispiel 7:** Enthaarungscreme auf Thioglykolsäurebasis

| | |
|---|---|
| 2,0 g | Dimethylisosorbit |
| 25,0 g | Calciumcarbonat |
| 6,5 g | Cetylstearylalkohol, selbstemulgierend |
| 5,0 g | Thioglykolsäure |
| 1,4 g | Lithiumhydroxid |
| 60,1 g | Wasser, vollentsalzt |
| 100,0 g | |

Die obige Enthaarungscreme besitzt einen pH-Wert von 12,5.

Die Enthaarungscreme wird mit einem Spatel so aufgetragen, daß alle zu entfernenden Haare gleichmäßig von ihr bedeckt werden. Nach einer Einwirkungszeit von etwa 5 Minuten wird die Enthaarungscreme mit einem Spatel oder einem Wattebausch entfernt. Anschließend wird die Haut zur Entfernung von Enthaarungsmittelresten mit 35 Grad Celsius warmem Wasser gründlich abgewaschen.

Alle in der vorliegenden Anmeldung enthaltenen Prozentangaben stellen Gewichtsprozente dar.

## Ansprüche

1. Kosmetisches Mittel zur dauerhaften Verformung oder Entfernung von Haaren auf der Basis einer keratinreduzierenden Mercaptoverbindung, dadurch gekennzeichnet, daß es Dimethylisosorbit der Formel (I)

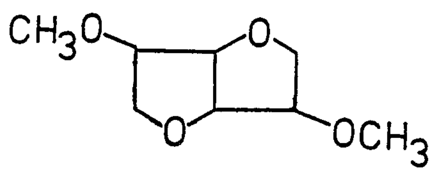

(I)

enthält.

2. Mittel nach Anspruch 1, dadurch gekennzeichnet, daß es 0,01 bis 5 Gewichtsprozent Dimethylisosorbit enthält.

3. Mittel nach Anspruch 1 oder 2, dadurch gekennzeichnet, daß die keratinreduzierende Mercaptoverbindung eine Mercaptocarbonsäure, ein Salz oder Ester einer Mercaptocarbonsäure, Cystein, Cysteamin, N-Acetyl-L-cystein, ein Sulfit oder ein Sulfid ist.

4. Mittel nach einem der Ansprüche 1 bis 3, dadurch gekennzeichnet, daß es die keratinreduzierende Mercaptoverbindung in einer Menge von 5 bis 30 Gewichtsprozent enthält.

5. Mittel nach einem der Ansprüche 1 bis 4, dadurch gekennzeichnet, daß es in Form eines Mittels zur dauerhaften Haarverformung vorliegt und einen pH-Wert von 4,5 bis 10 besitzt.

6. Mittel nach einem der Ansprüche 1 bis 4, dadurch gekennzeichnet, daß es in Form eines Mittels zur Entfernung der Haare vorliegt und einen pH-Wert von 11 bis 12,5 besitzt.

7. Verfahren zur dauerhaften Verformung von Haaren, bei dem man das Haar bevor und/oder nachdem man es in die gewünschte Form bringt mit einem Verformungsmittel behandelt, mit Wasser spült, sodann oxidativ nachbehandelt, mit Wasser spült, anschließend zur Frisur legt und sodann trocknet, dadurch gekennzeichnet, daß für die Verformungsbehandlung ein Mittel nach einem der Ansprüche 1 bis 5 verwendet wird.

8. Verfahren nach Anspruch 7, dadurch gekennzeichnet, daß man das Verformungsmittel 5 bis 45 Minuten auf das Haar einwirken läßt.

9. Verfahren nach Anspruch 7 oder 8, dadurch gekennzeichnet, daß man das Verformungsmittel in einer Menge von 80 bis 120 Gramm anwendet.

10. Verfahren zur Entfernung von Haaren, bei dem man ein Enthaarungsmittel auf die zu behandelnden Stellen aufbringt, das Enthaarungsmittel nach einer kurzen Einwirkungszeit wieder entfernt und anschließend mit lauwarmem Wasser nachspült, dadurch gekennzeichnet, daß für die Enthaarungsbehandlung ein Mittel nach einem der Ansprüche 1 bis 4 oder 6 verwendet wird

Europäisches Patentamt

# EUROPÄISCHER RECHERCHENBERICHT

Nummer der Anmeldung

EP 88 11 1186

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A,P | EP-A-0 261 387 (WELLA)<br>* ganzes Dokument *<br>--- | 1-10 | A 61 K 7/09<br>A 61 K 7/155 |
| A | PATENT ABSTRACTS OF JAPAN<br>Band 10, Nr. 373 (C-391)(2430), 12. Dezember 1986; & JP - A - 65 165 316 (KISHIYOUHIN) 26.07.1986<br>--- | 1-10 | |
| A | US-A-4 218 435 (SHIBA)<br>* Spalte 3, Tabelle 1; Ansprüche *<br>--- | 1-10 | |
| A | US-A-4 322 359 (HILLARD)<br>* Spalte 1, Ansprüche *<br>--- | 1-10 | |
| A | EP-A-0 205 770 (RÜTERSWERK)<br>* Spalte 1 *<br>--- | 1-10 | |
| A | CHEMICAL ABSTRACTS<br>Band 103, Nr. 4, 29. Juli 1985, Seite 301, Spalte 2, Zusammenfassungsnr. 27200x, Columbus, Ohio, US; S. BENNETT et al.: "Optimization of bioavailability of topical steroids: nonoccluded penetration enhancers under thermodynamic control", & J. PHARM. PHARMACOL. 1985, 37(5), 298-304<br>----- | 1-10 | RECHERCHIERTE SACHGEBIETE (Int. Cl.3)<br>A 61 K |

Der vorliegende Recherchenbericht wurde für alle Patentansprüche erstellt

| Recherchenort | Abschlußdatum der Recherche | Prüfer |
|---|---|---|
| BERLIN | 20-09-1988 | AVEDIKIAN P.F. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)